# FASCICULE DE BREVET EUROPEEN

(11) **EP 2 125 921 B1**
(45) Date de publication et mention de la délivrance du brevet: **24.08.2011**
(21) Numéro de dépôt: 08708293.9
(22) Date de dépôt: 28.01.2008
(51) Int. Cl.: C08F 293/00, C08F 2/38

(54) **PROCEDE DE PREPARATION DE PARTICULES DE MICROGEL PAR POLYMERISATION RADICALAIRE CONTROLEE EN DISPERSION AQUEUSE METTANT EN UVRE DES AGENTS DE CONTRÔLE NITROXYDES.**
VERFAHREN ZUR HERSTELLUNG VON MIKROGELPARTIKELN DURCH GESTEUERTE RADIKALE POLYMERISIERUNG IN EINER WÄSSRIGEN DISPERSION MIT NITROXID-STEUERUNGSMITTELN
METHOD FOR PREPARING MICROGEL PARTICLES BY CONTROLLED RADICAL POLYMERIZATION IN AN AQUEOUS DISPERSION USING NITROXIDE CONTROL AGENTS

(30) Priorité: 29.01.2007 FR 0752941
(43) Date de publication de la demande: 02.12.2009
(73) Titulaire: ARKEMA FRANCE, 92700 Colombes (FR); Centre National de la Recherche Scientifique, 75016 Paris (FR); Université Pierre et Marie Curie (Paris VI), 75252 Paris Cedex 05 (FR)
(72) Inventeur: MAGNET, Stéphanie, F-64370 Morlanne (FR); CHARLEUX, Bernadette, F-94000 Vincennes (FR); DELAITTRE, Guillaume, D-68165 Mannheim (FR); SAVE, Maud, F-65250 Escala (FR)
(74) Mandataire: Albani, Dalila
(86) Numéro de dépôt international: PCT/EP2008/050975
(87) Numéro de publication internationale: WO 2008/095814

(56) Documents cités:
- EP-A1- 1 000 954
- WO-A-01/77198
- WO-A-98/31739
- WO-A-99/58588

## Description

### DOMAINE TECHNIQUE

La présente invention a trait à un procédé de préparation de particules de microgel selon un procédé de polymérisation radicalaire contrôlée en dispersion aqueuse mettant en oeuvre des agents de contrôle, en particulier des agents de contrôle du type nitroxyde.

Les particules de microgel obtenues par ce procédé peuvent être utilisées en tant que matériau d'encapsulation, par exemple, pour des médicaments, des insecticides, des herbicides, des charges ou des pigments.

Elles peuvent être également utilisées en tant qu'additifs destinés à modifier la viscosité de certains milieux liquides.

Ainsi, les particules de microgel obtenues par ce procédé trouvent tout particulièrement leur intérêt dans le domaine de la pharmacie, du biomédical, de l'agrochimie, de l'alimentaire pour animaux et humains, de la cosmétique, des revêtements de surface tels que les peintures.

### ÉTAT DE LA TECHNIQUE ANTÉRIEURE

Des procédés de préparation de particules de microgel ont déjà fait l'objet d'études dans l'art antérieur.

Les particules de microgel sont réalisées classiquement en dispersion aqueuse par un procédé de polymérisation radicalaire conventionnelle (c'est-à-dire une polymérisation non vivante) mettant en oeuvre des monomères vinyliques éventuellement en présence d'un ou plusieurs comonomères réticulants.

Une polymérisation en dispersion aqueuse se caractérise par le fait qu'en début de polymérisation, le milieu réactionnel est une solution homogène (les monomères étant solubles), alors qu'en fin de polymérisation, le milieu réactionnel se présente sous la forme d'un latex, c'est-à-dire un ensemble de particules de polymère stabilisées dans le milieu réactionnel.

Principalement, trois protocoles de polymérisation radicalaire conventionnelle en dispersion aqueuse ont été mis en oeuvre dans l'art antérieur pour la réalisation de particules de microgel :
- un premier protocole mettant en oeuvre la présence d'additifs tensioactifs (tel que le laurylsulfate de sodium). Le rôle de l'additif tensioactif est d'assurer la stabilité colloïdale du latex. Les tailles de particules de microgel obtenues sont de l'ordre ou sont inférieures à une centaine de nanomètres. Cependant, en fin de polymérisation, une étape d'élimination du tensioactif doit être envisagée ;
- un deuxième protocole mettant en oeuvre un amorceur de polymérisation radicalaire chargé et ceci en l'absence d'additifs tensioactifs. La stabilité colloïdale du latex est alors assurée par la présence de fragments de l'amorceur chargés qui sont situés en extrémité de chaînes. Cependant, afin d'assurer une bonne stabilité du latex final, le taux de solides de l'émulsion doit rester très faible et généralement inférieur à 5% en poids ;
- un troisième protocole mettant en jeu un comonomère ionique, ionisable ou hydrosoluble non chargé et ne nécessitant pas l'utilisation d'additifs tensioactifs. Dans ce cas, le comportement thermique du latex est très sensible à la composition du système.

D'autres auteurs ont mis en place des procédés de préparation de particules de microgel par le biais d'une polymérisation radicalaire contrôlée. Ainsi WO 2001077198 décrit un procédé de préparation de micelles réticulées formant des particules de microgel faisant intervenir une polymérisation radicalaire contrôlée du type « RAFT » (correspondant à la terminologie anglaise « Reversible addition-fragmentation chain transfer ») comprenant les étapes suivantes :
- une étape de polymérisation RAFT en présence d'un agent de transfert de chaîne du type RAFT d'un ou plusieurs monomères solvophobes et d'un ou plusieurs monomères solvophiles pour former un copolymère à blocs comprenant un ou plusieurs blocs solvophobes et un ou plusieurs blocs solvophiles, le ou lesquels blocs solvophobes sont insolubles dans un milieu de dispersion et le ou lesquels blocs solvophiles étant solubles dans un milieu de dispersion ;
- une étape de dispersion dudit copolymère à blocs dans ledit milieu de dispersion pour former des micelles ; et
- une étape de stabilisation des micelles pour former ledit microgel.

L'étape de stabilisation peut être mise en oeuvre par réticulation d'une ou plusieurs fonctions présentes sur les blocs du copolymère à blocs.

Ce procédé présente les inconvénients suivants :
- il est mis en oeuvre dans un milieu comprenant un solvant organique, ce qui peut nécessiter des étapes de post-traitement afin de pouvoir utiliser ces particules dans des domaines d'application proscrivant les solvants organiques ;
- il nécessite un grand nombre d'étapes et de manipulations différentes, de mise en oeuvre pas toujours aisée ;
- dans le cas de la polymérisation RAFT, l'utilisation d'un amorçage conventionnel peut occasionner la formation d'homopolymères, les conditions d'obtention de copolymères à blocs n'étant pas faciles à déterminer.

Il existe donc un véritable besoin pour un procédé de préparation de particules de microgel, qui puisse être mis en oeuvre en milieu aqueux, en l'absence d'additifs tensioactifs, et qui permette l'obtention de dispersions stables et concentrées de particules de microgel de morphologie contrôlée et dont les tailles de particules peuvent être égales ou inférieures à 100 nanomètres et qui soit de mise en oeuvre simple.

### EXPOSÉ DE L'INVENTION

Ainsi, l'invention a trait à un procédé de préparation de particules de microgel en milieu aqueux comprenant les étapes suivantes :
a) une étape de préparation d'un macroamorceur vivant obtenu par polymérisation d'un ou plusieurs monomères en présence d'un agent de contrôle répondant à l'une des formules suivantes : 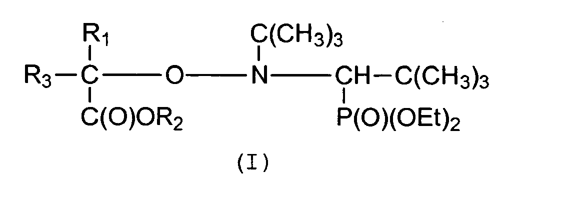 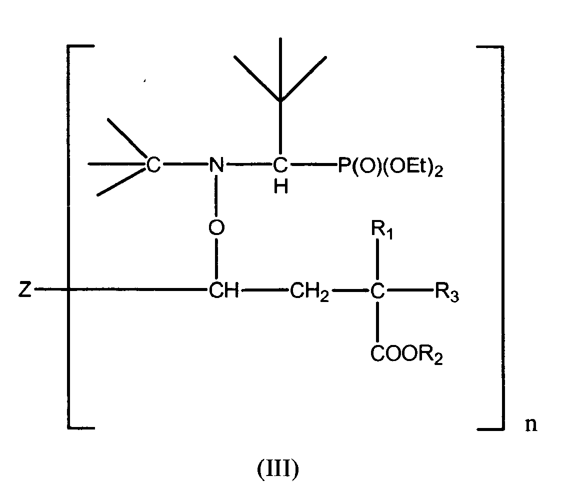 dans lesquelles :
   * R₁ et R₃, identiques ou différents, représentent un groupe alkyle, linéaire ou ramifié, ayant un nombre d'atomes de carbone allant de 1 à 3 ;
   * R₂ représente un atome d'hydrogène, un groupe alkyle, linéaire ou ramifié, ayant un nombre d'atomes de carbone allant de 1 à 8, un groupe phényle, un métal alcalin tel que Li, Na, K, un ion ammonium tel que NH₄⁺, NHBu₃⁺ ; de préférence R₁ et R₃ étant CH₃ et R₂ étant H ;
   * Z représente un groupe aryle ou un groupe de formule Z₁-[X-C(O)]ₙ, dans laquelle Z₁ représente une structure polyfonctionnelle provenant par exemple d'un composé du type polyol, X est un atome d'oxygène, un atome d'azote porteur d'un groupement carboné ou d'un atome d'hydrogène, ou un atome de soufre ; et
      - n est un nombre entier supérieur ou égal à 2 ;
b) une étape d'ajout au milieu réactionnel obtenu à l'issue de l'étape a) d'au moins un monomère vinylique, lequel monomère étant différent de celui de l'étape a) ;
c) une étape d'ajout au milieu réactionnel d'au moins un comonomère réticulant, le comonomère réticulant étant différent des monomères des étapes a) et b), cette étape d'ajout c) étant réalisée simultanément ou en différé par rapport à l'étape d'ajout b),
dans lequel le ou les monomères constitutifs du macroamorceur vivant de l'étape a) et/ou les monomères vinyliques de l'étape b) et/ou le ou les comonomères réticulants de l'étape c) comprennent au moins une fonction choisie parmi -CO₂H, -SO₃H, ammonium, oxyde d'éthylène, amino, amide, ladite fonction pouvant exister éventuellement sous forme de sels.

On entend généralement par groupe amino, un groupe -NH₂ éventuellement N-substitué(s), par exemple, par un ou plusieurs groupes alkyles.

On entend généralement par groupe amide un groupe -CO-NH₂ éventuellement N-substitué(s), par exemple, par un ou plusieurs groupes alkyles.

On entend généralement par groupe ammonium, un groupe -NH₃⁺, éventuellement N-substitué(s), par exemple, par un ou plusieurs groupes alkyles.

On précise que l'abréviation Et correspond au groupe éthyle et que l'abréviation Bu correspond au groupe butyle, lequel peut exister sous différentes formes isomères (n-butyle, sec-butyle, tert-butyle).

Le procédé de l'invention se distingue essentiellement des procédés de l'art antérieur par l'utilisation d'un agent de contrôle du type nitroxyde tel que défini ci-dessus, qui permet la formation d'un macroamorceur vivant à partir duquel peut être réengagée une réaction de polymérisation avec des monomères vinyliques et un ou plusieurs comonomères réticulants, le choix de ces monomères et comonomères étant effectué de façon à générer des particules de microgel présentant les propriétés voulues.

Une des caractéristiques importantes de ce procédé est que les monomères entrant dans la constitution des particules de microgel (monomères du macroamorceur vivant et/ou monomères vinyliques et/ou comonomères réticulants) comprennent au moins une fonction -CO₂H, -SO₃H, ammonium, oxyde d'éthylène, amino, amide. Ces fonctions remplissent en milieu aqueux un rôle de stabilisant. Il est ainsi possible de s'affranchir dans le milieu réactionnel de l'ajout d'additifs tensioactifs, comme cela était le cas de l'art antérieur, et par voie de conséquence, des étapes d'élimination de ces additifs tensioactifs en fin de procédé. Les particules ainsi obtenues présentent une stabilité inhérente aux monomères les constituant.

Le procédé de l'invention permet également, en jouant notamment sur la teneur en comonomère réticulant, de contrôler la taille ainsi que la morphologie des particules obtenues. Il est ainsi possible d'obtenir des particules présentant une taille inférieure ou égale à 100 nm.

Le procédé de l'invention se déroule classiquement dans un seul milieu réactionnel et ne nécessite pas comme c'est le cas de certains procédés de l'art antérieur une mise en dispersion dans un milieu de dispersion approprié de particules préalablement synthétisées.

Du fait que le procédé de l'invention se déroule en milieu aqueux, les particules obtenues en fin de procédé peuvent être utilisées telles quelles, à savoir sans nécessiter d'étapes de post-traitement.

Qui plus est, par rapport à une polymérisation du type RAFT, la présence d'homopolymères est limité, car seul le premier bloc (c'est-à-dire le macroamorceur vivant) crée des radicaux et donc amorce la polymérisation.

Selon l'invention, on précise que par particules de microgel, on entend généralement des particules polymériques en suspension dans le milieu aqueux, présentant une taille inférieure ou égale à 100 nm, de préférence allant de 40 à 300 nm.

On précise que par macroamorceur vivant, on entend, au sens de l'invention, un polymère comprenant au moins une extrémité apte à être réengagée dans une réaction de polymérisation par ajout de monomères dans le milieu réactionnel. Le terme « macroamorceur vivant » correspond aussi à la terminologie « polymère vivant ».

On précise que, par monomère vinylique, on entend des monomères comprenant une fonction éthylénique apte à réagir dans une réaction de polymérisation.

De préférence, les monomères constitutifs du macroamorceur vivant de l'étape a) comprennent au moins une fonction choisie parmi -CO₂H, -SO₃H, ammonium, oxyde d'éthylène, amino, amide, ladite fonction pouvant exister éventuellement sous forme de sels.

Des monomères constitutifs du macroamorceur vivant de l'étape a) peuvent être choisis parmi les monomères (méth)acryliques, les monomères (méth)acrylates de dialkylaminoalkyle, les halogénures de (méth)acrylates de trialkylammoniumalkyle, les monomères (méth)acrylates d'(alcoxy)polyalkylèneglycol, les monomères (méth)acrylamides, comme les (di)alkyl(méth)acrylamides, comprenant éventuellement un groupe sulfonate (tel que l'acrylamidométhylpropanesulfonate), les monomères styréniques comprenant un groupe sulfonate (tels que le styrène sulfonate).

On peut citer avantageusement dans la famille des monomères carboxyliques insaturés l'acide acrylique ou l'acide méthacrylique.

A titre d'exemple d'agent de contrôle susceptible d'être utilisé, on peut citer celui répondant à la formule suivante :

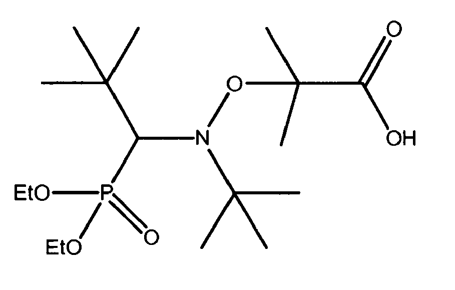

Les agents de contrôle de formule (III) sont issus généralement d'un procédé consistant à faire réagir une ou plusieurs alcoxyamines de formule (I) suivante : 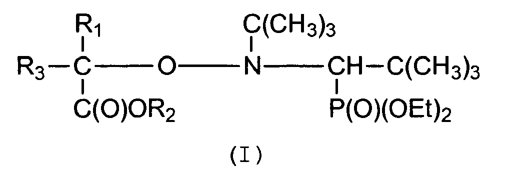 dans laquelle R₁, R₂ et R₃ sont tels que définis précédemment,
avec au moins un composé polyinsaturé de formule (II) : 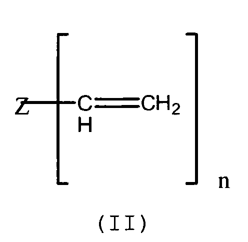 dans laquelle Z représente un groupement aryle ou un groupe de formule Z₁- [X-C (O)]ₙ dans laquelle Z₁ représente une structure polyfonctionnelle provenant par exemple d'un composé du type polyol, X est un atome d'oxygène, un atome d'azote porteur d'un groupement carboné ou d'un atome d'hydrogène, ou un atome de soufre et n est un nombre entier supérieur ou égal à 2,
en présence ou non de solvant(s), de préférence choisi(s) parmi les alcools comme l'éthanol, les solvants aromatiques, les solvants chlorés, les éthers et les solvants polaires aprotiques,
à une température allant, en général, de 0 à 90°C, de préférence de 25 à 80°C,
le ratio molaire entre alcoxyamine(s) monofonctionnelle(s) de formule (I) et composé(s) polyinsaturé(s) de formule (II) allant de 1,5 à 1,5 n, de préférence, de n à 1,25 n, n étant tel que défini ci-dessus.

A titre d'exemples de composés polyinsaturés utilisables pour réaliser des alcoxyamines polyfonctionnelles telles que définies ci-dessus, on peut citer les vinylbenzènes polyfonctionnels (Z étant alors un groupe aryle) ou parmi les dérivés acryliques polyfonctionnels (Z étant alors un groupe de formule Z₁-[X-C(O)]ₙ). De préférence, le composé polyinsaturé est le divinylbenzène, le trivinylbenzène, l'éthylène glycol diacrylate, le 1,3-butanediol diacrylate, le 1,4-butanediol diacrylate, le 1,6-hexanediol diacrylate, le néopentyl glycol diacrylate, le cyclohexane diméthanol diacrylate, le diéthylène glycol diacrylate, le triéthylène glycol diacrylate, le tétraéthylène glycol diacrylate, le dipropylèneglycol diacrylate, le tripropylèneglycol diacrylate, les polyéthylène glycol diacrylates (commercialisés par Sartomer sous les dénominations SR259, SR344, SR610), les hexanediol diacrylates alcoxylés (commercialisés par Sartomer sous les dénominations CD561, CD564, CD560), le bisphénol-A diacrylate, les bisphénol-A diacrylate éthoxylés (commercialisés par Sartomer sous les dénominations SR 349, SR601, SR602, CD9038), le triméthylolpropane triacrylate, le pentaérythritol triacrylate, le tris(2-hydroxyéthyl)isocyanurate triacrylate, les triméthylolpropane triacrylate éthoxylés (commercialisés par Sartomer sous les dénominations SR454, SR499, SR502, SR9035, SR415), le glycéryl triacrylate propoxylé (commercialisé par Sartomer sous la dénomination SR9020), les triméthylolpropane triacrylate propoxylés (commercialisés par Sartomer sous les dénominations SR492 et CD501), le pentaérythritol tétraacrylate, le di-triméthylolpropane tétracrylate, le pentaérythritol tétraacrylate éthoxylé (commercialisé par Sartomer sous la dénomination SR494), le dipentaérythritol pentacrylate, les caprolactones modifiées dipentaérythritol hexaacrylate (commercialisés par Sartomer sous les dénominations Kayarad DCPA20 et DCPA60), le dipentaérythritol polyacrylate (commercialisé par UCB Chemicals sous la dénomination DPHPA).

Lorsque Z correspond à la formule Z₁-[X-C(O)]ₙ, les agents de contrôle répondent à la formule (IIIa) suivante : 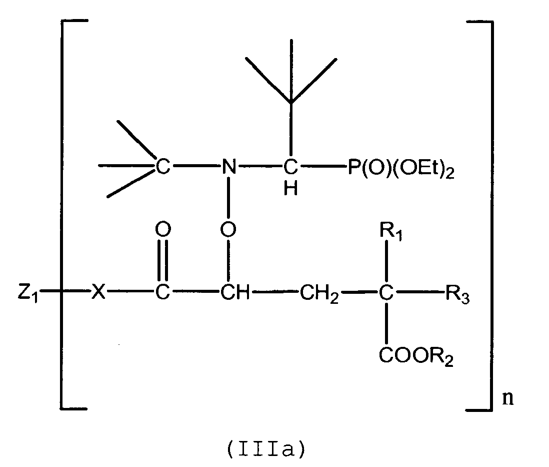 Z₁ correspondant généralement à un groupe alkylène.

Un exemple particulier d'agent de contrôle conforme à la définition générale donnée ci-dessus est l'alcoxyamine polyfonctionnelle répondant à la formule suivante : 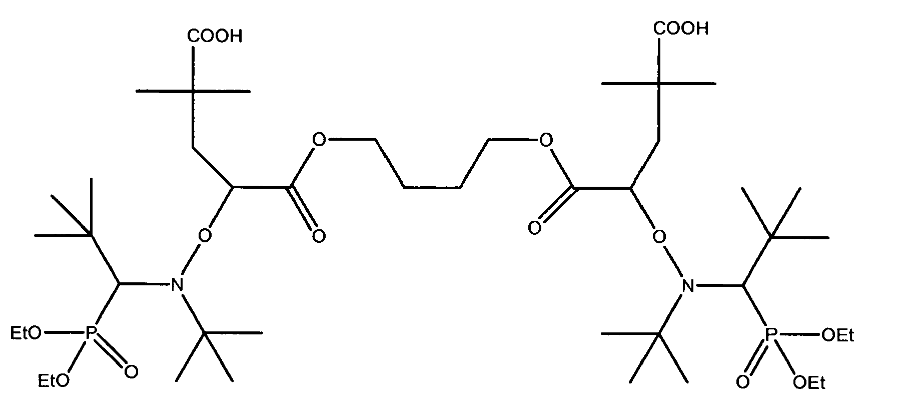 cette alcoxyamine polyfonctionnelle étant issue de la réaction d'une alcoxyamine monofonctionnelle de formule (I) avec du 1,4-butanediol diacrylate.

Les agents de contrôle de formule (I) et/ou les agents de contrôle de formule (III) jouent également un rôle d'agent initiateur et d'agent émulsifiant; ainsi, les propriétés tensioactives des agents de contrôle de formule (I) et/ou agents de contrôle de formule (III) permettent d'éviter ainsi l'emploi d'additifs tensioactifs.

Qui plus est, certains des monomères entrant dans la constitution des particules comprenant des fonctions -CO₂H, -SO₃H, ammonium, oxyde d'éthylène, amino, amide contribuent également à stabiliser lesdites particules sans nécessiter l'emploi de tensioactifs.

A l'issue de l'étape a), l'on obtient ainsi un macroamorceur vivant comprenant une extrémité de formule suivante : 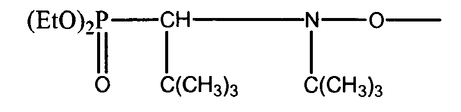 cette extrémité étant désignée par l'abréviation SG1.

Ce macroamorceur comprenant une telle extrémité réactive peut être réengagé dans une réaction de polymérisation, moyennnant l'ajout de monomères, ce qui est le cas de l'invention dans le cadre des étapes b) et c).

Les monomères vinyliques introduits dans le cadre de l'étape b) peuvent être choisis parmi les monomères vinylaromatiques tels que le styrène ou les styrènes substitués, les monomères acryliques tels que les acrylates d'alkyle ou d'aryle, les acrylates fonctionnels, les monomères méthacryliques tels que les méthacrylates d'alkyle ou d'aryle, les méthacrylates fonctionnels et les monomère de la famille des acrylamides et des méthacrylamides.

De préférence, les monomères vinyliques introduits dans le cadre de l'étape b) sont des monomères de la famille des acrylamides et des méthacrylamides.

De préférence, les monomères acrylamides sont des monoalkylacrylamides ou des dialkylacrylamides.

A titre d'exemple, on peut citer comme monomère dialkylacrylamide utilisable, le N,N-diméthylacrylamide, le N,N-diéthylacrylamide ou encore le N,N-diisopropylacrylamide.

A titre d'exemple, on peut citer, comme monomère monoalkylacrylamide utilisable le N-méthylacrylamide, le N-éthylacrylamide, le N-isopropylacrylamide.

Ces monomères vinyliques sont introduits, avantageusement, dans le milieu réactionnel, à raison d'au moins 10% en poids total du milieu réactionnel, et de préférence supérieur à 20% en poids.

La particularité des polymères présentant des unités acrylamides est qu'ils présentent une température critique de solution la plus basse (LCST) dans l'eau (aux alentours de 32°C pour les unités issues du N-isopropylacrylamide). Au-dessous de cette température, le polymère est soluble dans le milieu réactionnel, alors qu'il précipite pour des températures supérieures à la LCST. Ces modifications d'état physique du polymère en fonction de la température confèrent aux particules de microgel des propriétés d'encapsulation. En effet, lorsque le polymère est chauffé à des températures supérieures à la LCST et en présence d'un principe actif, ce dernier est encapsulé dans la particule, et est ensuite relargué à basse température (plus précisément à une température inférieure à la LCST).

L'inconvénient des polymères à LSCT est que les particules se dissolvent selon les conditions de température, dans lesquelles les polymères sont placés. L'invention comprenant une étape d'ajout d'un comonomère réticulant permet aux particules de conserver leur intégrité même à des températures inférieures à la LCST et d'éviter la solubilisation du polymère dans le milieu réactionnel.

Selon l'invention, le procédé comprend une étape c) consistant à ajouter un comonomère réticulant.

Par comonomère réticulant, on entend un monomère qui, de par sa réactivité avec les autres monomères présents dans le milieu de polymérisation, est capable de générer un réseau tridimensionnel enchevêtré. D'un point de vue chimique, un comonomère réticulant comprend généralement au moins deux fonctions polymérisables éthyléniques, qui en réagissant, sont susceptibles de créer des ponts entre plusieurs chaînes polymériques.

Ces comonomères réticulants peuvent être susceptibles de réagir avec les monomères insaturés de l'étape b).

Parmi les comonomères réticulants, on peut citer les divinylbenzènes, les trivinylbenzènes, les (méth)acrylates allyliques, les (méth)acrylates de diallylmaléatepolyol tels que les tri(méth)acrylates de triméthylolpropane, les di(méth)acrylates d'alkylèneglycol qui ont de 2 à 10 atomes de carbone dans la chaîne carbonée tels que les di(méth)acrylates d'éthylèneglycol, les di(méth)acrylates de 1,4-butanediol, les di(méth)acrylates de 1,6-hexanediol, les N,N'-alkylène bisacrylamides, tels que le N,N'-méthylène bisacrylamide. De préférence, on utilisera comme agent réticulant le N,N'-méthylène bisacrylamide.

Les comonomères réticulants peuvent être également des composés qui sont susceptibles de réagir avec les produits de polymérisation. On peut citer les diglycidyl éthers, tels que l'éthylène glycol diglycidyl éther.

Le comonomère réticulant est introduit, avantageusement, dans le milieu réactionnel, en une teneur allant de 1 à 12% en poids par rapport au poids de monomère(s) vinylique(s) introduit(s) dans l'étape b) ou de 0,2 à 2% en poids par rapport à l'ensemble du milieu réactionnel.

L'étape d'ajout c) peut être réalisée simultanément ou en différé par rapport à l'étape d'ajout b).

Ainsi, selon un premier mode de réalisation, le comonomère réticulant peut être ajouté en même temps que le(s) monomère(s) vinyliques. Ce mode de réalisation est particulièrement approprié, lorsque le comonomère réticulant est introduit en une quantité inférieure à 10% en poids, plus particulièrement inférieure à 5% en poids par rapport à celle du(des) monomère(s) vinylique(s). Le caractère contrôlé de la polymérisation radicalaire permet d'obtenir une modération au niveau de la croissance de chaînes et d'éviter ainsi la création de noeuds de réticulation en solution avant la micellisation.

Selon un second mode de réalisation, le comonomère réticulant peut être ajouté en différé par rapport au(x) monomère(s) vinylique(s). De préférence, le comonomère réticulant est introduit après l'étape de formation des particules de polymères (dite étape de nucléation). Ce mode de réalisation est particulièrement approprié, lorsque le comonomère réticulant est introduit en une quantité supérieure à 5% en poids par rapport à celle du(des) monomère(s) vinylique(s), plus particulièrement supérieure à 10% en poids. En différant de la sorte l'addition de comonomère réticulant, on s'affranchit de la formation de macrogel dans le milieu réactionnel. Il est alors possible d'augmenter le dosage du comonomère réticulant et de régler ainsi le niveau de réticulation de la particule en son coeur. Ce mode de réalisation est particulièrement avantageux, en ce sens qu'il permet de dissocier l'étape de nucléation de l'étape de réticulation, afin d'obtenir un meilleur contrôle des tailles de particules générées au cours du procédé. Du fait de leur caractère vivant, toutes les chaînes polymériques sont incorporées dans le microgel.

Que ce soit pour les modes de réalisation décrits ci-dessus ou d'autres modes, le procédé de l'invention est mis en oeuvre, généralement, à des températures généralement supérieures à 80°C et de préférence supérieures à 100°C.

Le procédé de l'invention est mis en oeuvre en milieu aqueux et, avantageusement, en l'absence d'additifs tensioactifs. En effet, une des particularités de l'invention est que la stabilité colloïdale des particules de microgel obtenue n'est pas assurée par l'adjonction d'additifs tensioactifs au milieu réactionnel, mais est assurée par la présence de polymères comprenant des fonctions tensioactives, en particulier des fonctions -CO₂H.

La concentration en matières actives, c'est-à-dire polymères et monomères résiduels, dans le milieu de polymérisation est, avantageusement, supérieure à 10% en poids, et de préférence supérieure à 20% en poids.

Lorsque les particules de microgel préparées selon le procédé de l'invention sont destinées à constituer des matériaux d'encapsulation, il peut être envisageable de prévoir dans le cadre du procédé une étape d'ajout au milieu réactionnel des éléments destinés à être encapsulés. Cette étape d'ajout peut être simultanée à l'étape b) ou si l'étape c) est différée par rapport à l'étape b), cette étape d'ajout peut se dérouler simultanément ou après l'étape c).

Les éléments destinés à être encapsulés peuvent être des molécules organiques, telles que des médicaments, des insecticides, des herbicides, des colorants, des molécules inorganiques telles que des pigments inorganiques, des catalyseurs, des charges minérales.

Enfin, le procédé de l'invention peut comprendre, si besoin est, une étape d'isolement des particules de microgel du milieu aqueux, cette étape d'isolement pouvant être réalisée, par exemple, par filtration.

L'invention a également pour objet des particules de microgel susceptibles d'être obtenues par le procédé tel que défini ci-dessus.

Les particules de microgel obtenues selon le procédé de l'invention présentent un coeur plus ou moins réticulé, selon la teneur en comonomère réticulant utilisé, et une couronne chevelue, qui leur confère une bonne stabilité sous forme de colloïdes, quelle que soit la température, l'hydrophilie ou l'hydrophobie du coeur. La nature des monomères utilisés qui constituent le coeur de la particule pourra être modulée en fonction de la propriété que l'on souhaite conférer à la particule de microgel. Par exemple, si l'on souhaite obtenir des particules de microgel thermosensibles, on choisira d'utiliser des monomères du type dialkylacrylamide. Si l'on prend comme exemple, le monomère N,N-diéthylacrylamide, on observera le phénomène suivant : à haute température (à savoir, à une température supérieure à la LCST), les chaînes de copolymère à blocs s'assemblent et des particules de microgel hydrophobes sont formées. Lorsque la température du milieu réactionnel est inférieure à la LCST, les particules se gonflent d'eau mais ne se dissocient pas en raison de l'existence de noeuds de réticulation chimique et la viscosité du milieu augmente fortement.

De part leurs propriétés intrinsèques, les particules de microgel de l'invention obtenues selon le procédé de l'invention peuvent trouver des applications dans des domaines d'utilisation variés.

Les particules de microgel peuvent être notamment utilisées pour encapsuler des éléments tels que des molécules organiques, telles que des médicaments, des insecticides, des herbicides, des colorants, des molécules inorganiques telles que des pigments inorganiques, des catalyseurs, des charges minérales. Lorsque les particules sont à base d'unités LSCT, le relargage des éléments est alors possible, lorsque la température diminue et passe en-dessous de la LCST du polymère composant de la particule de microgel. Elles trouvent ainsi leur application tout particulièrement dans le domaine de la pharmacie, de l'agrochimie, de l'alimentaire pour animaux et humains, du biomédical, de la cosmétique, de la peinture.

Ces particules peuvent être aussi utilisées comme épaississants, afin de modifier la viscosité d'un milieu liquide, notamment dans le domaine des revêtements de surface.

Dans le domaine de la cosmétique, elles peuvent être utilisées dans des produits du type rouge à lèvres, anti-cernes ou encore fonds de teint. Ces particules peuvent en outre être la base de formulations du type huile dans phase gélifiée ou inversement ou encore du type phase aqueuse (gélifiée ou non) dans huile dans phase gélifiée.

L'invention va maintenant être décrite à l'aide des exemples suivants donnés à titre illustratif et non limitatif.

### BRÈVE DESCRIPTION DES DESSINS

Les figures 1 et 2 représentent respectivement le suivi de la conversion des monomères en polymère en fonction du temps et l'évolution de la taille des particules de microgel au cours de cycles de température dans le cadre de l'exemple 1.
Les figures 3 et 4 représentent respectivement le suivi de la conversion des monomères en polymère en fonction du temps et l'évolution de la taille des particules de microgel au cours de cycles de température dans le cadre de l'exemple 2.
Les figures 5 et 6 représentent respectivement le suivi de la conversion des monomères en polymère en fonction du temps et l'évolution de la taille des particules de microgel au cours de cycles de température dans le cadre de l'exemple 3.

### EXPOSÉ DÉTAILLÉ DE MODES DE RÉALISATION PARTICULIERS

### EXEMPLE 1

Cet exemple décrit la copolymérisation en dispersion aqueuse du N,N-diéthylacrylamide avec le N,N'-méthylène bisacrylamide, le N,N'-méthylène bisacrylamidè étant introduit dans le milieu réactionnel en même temps que le N,N-diéthylacrylamide à raison de 3% molaire de N,N'-méthylène bisacrylamide.

Dans un bécher sont dissous 1,61 g de macroamorceur poly(acide acrylique)-SG1 de masse molaire 1880 g.mol⁻¹, 0,45 g de Na₂CO₃ dans 18,52 g de NaOH 1M et 104,19 g d'eau permutée. Dans un autre bécher, 30,01 g de N,N-diéthylacrylamide sont mélangées à 1,09 g de N,N'-méthylène bisacrylamide et 0,0297 g de SG1. Les deux solutions sont ensuite réunies puis dégazées par bullage d'azote et agitées à 300 tours/min pendant 20 minutes.

Le mélange est ensuite introduit dans une cuve de 300 mL préalablement chauffée à 110°C puis mis sous agitation à 300 tours/min sous 3 bars de pression.

La température du milieu réactionnel est maintenue à 112°C. Un échantillon d'environ 5 mL est prélevé toutes les demi-heures puis toutes les heures pendant 8 heures et la réaction est stoppée après 24 heures.

La taille de particules est déterminée avant refroidissement de chaque échantillon en ajoutant quelques dizaines de µL de l'échantillon à 6 mL d'eau permutée à une température supérieure à 70°C dans une cuve parallélépipédique en polystyrène. L'échantillon dilué est ensuite analysé en Diffusion Dynamique de la Lumière sur un appareil Malvern Nano ZS90. L'échantillon dilué est ensuite caractérisé à froid en abaissant la consigne de température du Nano ZS90 à 15°C puis des cycles en température 50°C-15°C-50°C sont effectués.

Le taux de conversion des monomères en polymère est déterminé par gravimétrie. Dans une coupelle en aluminium, environ 1 g de chaque échantillon est déposé puis mis à évaporer sous une hotte toute une nuit puis quelques heures dans un dessicateur chauffant à 85°C sous pression réduite.

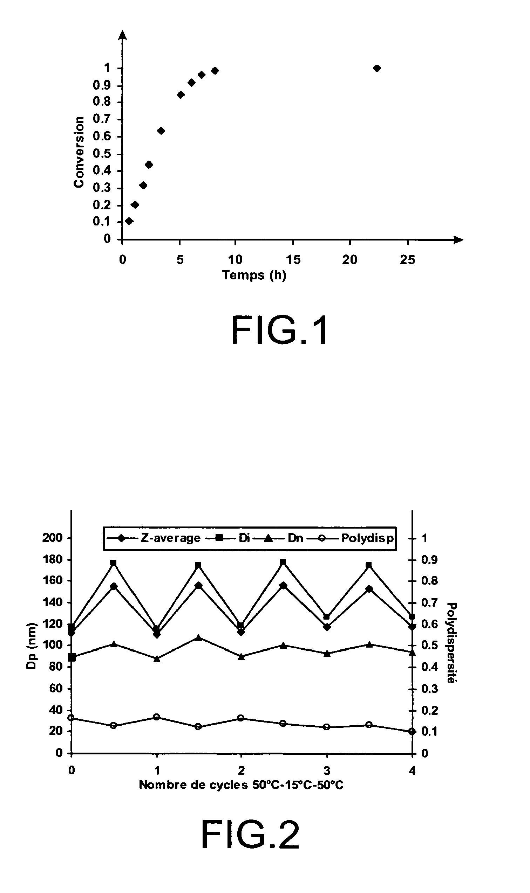

Les deux graphiques représentés sur les figures 1 et 2 présentent respectivement le suivi de la conversion des monomères en polymère et l'évolution de la taille des particules de microgels au cours des cycles de température.

Il apparaît sur la figure 1 sur la figure 1 que les monomères sont presque entièrement consommés en polymère après un peu moins d'une dizaine d'heures de réaction. Le latex est récupéré à chaud après pratiquement 24 heures et analysé avant refroidissement en-dessous de la LCST en Diffusion Dynamique de la Lumière. Sur la figure 2, on peut suivre l'évolution de la taille des particules en fonction de la température de mesure (15°C ou 50°C). Z-average est la moyenne calculée directement par l'appareil de Diffusion Dynamique de la Lumière Nano ZS90. Ce dernier donne également des moyennes en intensité (Dᵢ), en nombre (Dₙ) ainsi qu'un indice de polydispersité. Un échantillon est considéré comme monodisperse pour des valeurs de cet indice inférieur ou égal à 0,1. Il apparaît sur cette figure, que le diamètre des particules formées dans le réacteur (cycle 0) est d'environ 110 nm(Z-average). Le latex ensuite refroidi à 15°C (donc en-dessous de la LCST du poly(N,N-diéthylacrylamide)) présente une taille de particules plus élevée (environ 180 nm) dû à la solvatation par l'eau des chaînes de polymère du coeur redevenues hydrophiles. Puis si on réchauffe à 50°C, on retrouve la même taille de particules qu'à la sortie du réacteur, preuve que la réticulation est effective. De plus, la figure 2 montre que le phénomène est parfaitement réversible, puisque les tailles de particules à 15°C et 50°C mesurées alternativement ne présentent pas de dérive et restent constantes. De plus, le latex obtenu est proche de la limite de monodispersité.

### EXEMPLE 2

Cet exemple décrit la copolymérisation en dispersion aqueuse du N,N-diéthylacrylamide avec le N,N'-méthylène bisacrylamide, le N,N'-méthylène bisacrylamide étant introduit dans le milieu réactionnel en différé par rapport au N,N-diéthylacrylamide avec 3% molaire de N,N'-méthylène bisacrylamide.

Dans un bécher sont dissous 1,61 g de macroamorceur poly(acide acrylique)-SG1 de masse molaire 1880 g.mol⁻¹, 12,82 g de NaOH et 72,15 g d'H₂O permutée. Dans un autre bécher, sont mélangés 0,0205g de SG₁ et 20,78 g de N,N-diéthylacrylamide. Les deux solutions sont réunies en une solution 1. Une solution 2 est préparée en mélangeant 5,60g de NaOH, 32,05 g d'eau permutée, 0,0091g de SG1, 9,23 g de N,N-diéthylacrylamide et 1,09 g de N,N'-méthylènebisacrylamide.

Les solutions 1 et 2 sont dégazées par bullage d'azote et agitées à 300 tours/min pendant 20 minutes.

La solution 1 est introduite dans une cuve de 300 mL préalablement chauffée à environ 110°C puis mis sous agitation à 300 tours/min sous 3 bars. La température du milieu est maintenue à 112°C. Des échantillons d'environ 5 mL sont prélevés à 30 et 60 minutes. Puis après 1 heure de réaction, la solution 2 est introduite dans la cuve.

Des échantillons d'environ 5 mL sont prélevés toutes les demi-heures pendant encore 2 heures puis toutes les heures pendant 5 heures et enfin la réaction est stoppée après 24 heures.

La taille de particules est déterminée avant refroidissement de chaque échantillon en ajoutant quelques dizaines de µL de l'échantillon à 6 mL d'eau permutée à une température supérieure à 70°C dans une cuve parallélépipédique en polystyrène. L'échantillon dilué est ensuite analysé en Diffusion Dynamique de la Lumière sur un appareil Malvern Nano ZS90. L'échantillon dilué est ensuite caractérisé à froid en abaissant la consigne de température du Nano ZS90 à 15°C puis des cycles en température 50°C-15°C-50°C sont effectués.

Le taux de conversion des monomères en polymère est déterminé par gravimétrie. Dans une coupelle en aluminium, environ 1 g de chaque échantillon est déposé puis mis à évaporer sous une hotte toute une nuit puis quelques heures dans un dessicateur chauffant à 85°C sous pression réduite.

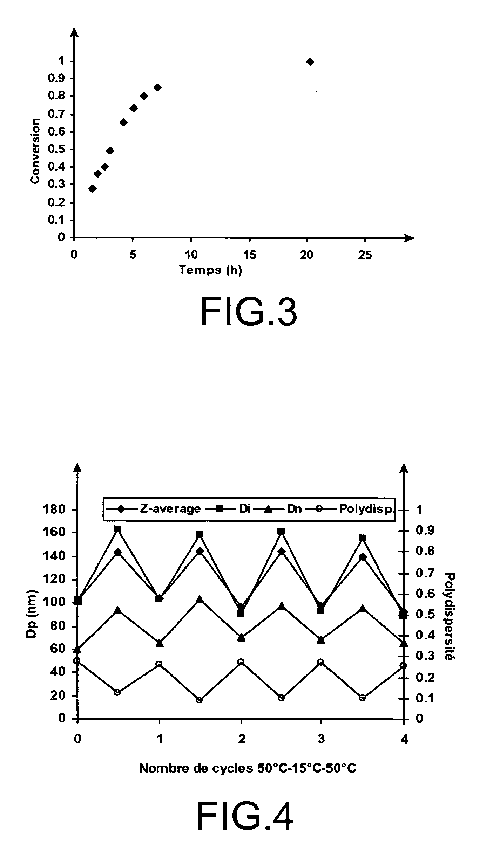

Les deux graphiques représentés sur les figures 3 et 4 présentent respectivement le suivi de la conversion des monomères en polymère et l'évolution de la taille des particules de microgel et de la polydispersité de la distribution au cours des cycles de température.

Il apparaît sur la figure 3 une conversion des monomères de 100% au bout d'une vingtaine d'heures.

La figure 4 montre un comportement similaire pour l'exemple 2 à celui de l'exemple 1 sauf que pour un même taux de réticulant, ici des diamètres de particule inférieures sont obtenus.

### EXEMPLE 3

Cet exemple décrit la copolymérisation en dispersion aqueuse du N,N-diéthylacrylamide avec le N,N'-méthylène bisacrylamide, le N,N'-méthylène bisacrylamide étant introduit dans le milieu réactionnel en différé par rapport au N,N-diéthylacrylamide avec 5% molaire de N,N'-méthylène bisacrylamide.

Dans un bécher sont dissous 1,73 g de macroamorceur poly(acide acrylique)-SG1 de masse molaire 2000 g.mol⁻¹, 17,28 g de NaOH et 92,69 g d'H₂O permutée. Dans un autre bécher, sont mélangés 0,0280 g de SG₁ et 27,76 g de N,N-diéthylacrylamide. Les deux solutions sont réunies en une solution 1. Une solution 2 est préparée en mélangeant 1,27 g de NaOH, 6,79 g d'eau permutée, 0,0021g de SG1, 2,05 g de N,N-diéthylacrylamide et 0,3644 g de N,N'-méthylènebisacrylamide.

Les solutions 1 et 2 sont dégazées par bullage d'azote et agitées à 300 tours/min pendant 20 minutes.

La solution 1 est introduite dans une cuve de 300 mL préalablement chauffée à environ 110°C puis mis sous agitation à 300 tours/min sous 3 bars. La température du milieu est maintenue à 112°C. Des échantillons d'environ 5 mL sont prélevés à 30 et 60 minutes. Puis après 1 heure de réaction, la solution 2 est introduite dans la cuve.

Des échantillons d'environ 5 mL sont prélevés toutes les demi-heures pendant encore 2 heures puis toutes les heures pendant 5 heures et enfin la réaction est stoppée après 24 heures.

La taille de particules est déterminée avant refroidissement de chaque échantillon en ajoutant quelques dizaines de µL de l'échantillon à 6 mL d'eau permutée à une température supérieure à 70°C dans une cuve parallélépipédique en polystyrène. L'échantillon dilué est ensuite analysé en Diffusion Dynamique de la Lumière sur un appareil Malvern Nano ZS90. L'échantillon dilué est ensuite caractérisé à froid en abaissant la consigne de température du Nano ZS90 à 15°C puis des cycles en température 50°C-15°C-50°C sont effectués.

Le taux de conversion des monomères en polymère est déterminé par gravimétrie. Dans une coupelle en aluminium, environ 1 g de chaque échantillon est déposé puis mis à évaporer sous une hotte toute une nuit puis quelques heures dans un dessicateur chauffant à 85°C sous pression réduite.

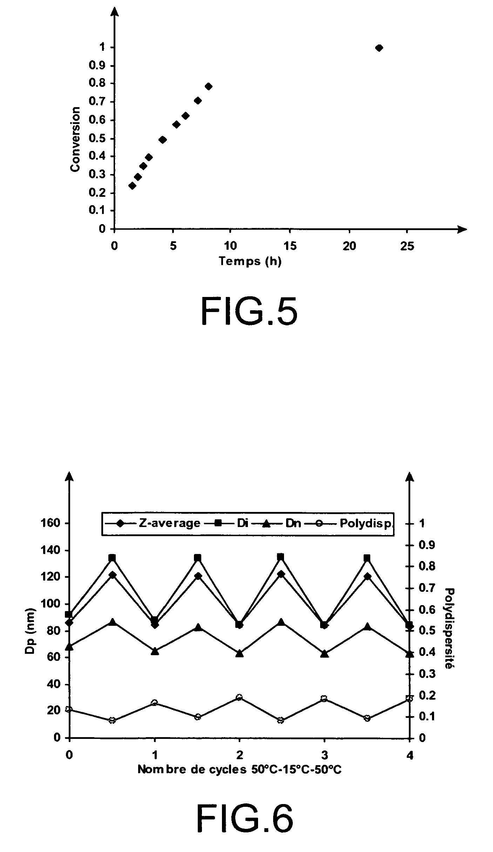

Les deux graphiques représentés sur les figures 5 et 6 présentent respectivement le suivi de la conversion des monomères en polymère et l'évolution de la taille des particules de microgel au cours des cycles de température.

La conversion en monomères atteinte après plus de 20 heures de réaction est de 100% comme le montre la figure 5. Cette fois encore, des particules de microgel thermosensibles sont obtenues et témoignent d'un caractère réversible de la sensibilité à la température et donc d'une réticulation effective. Les diamètres obtenus, comme le montre la figure 6, sont ici plus faibles que dans l'exemple 2 et la polydispersité à chaud diminue.

## Revendications

1. Procédé de préparation de particules de microgel en milieu aqueux comprenant les étapes suivantes :
a) une étape de préparation d'un macroamorceur vivant obtenu par polymérisation d'un ou plusieurs monomères en présence d'un agent de contrôle répondant à l'une des formules suivantes : 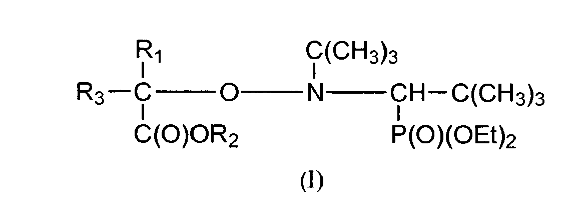 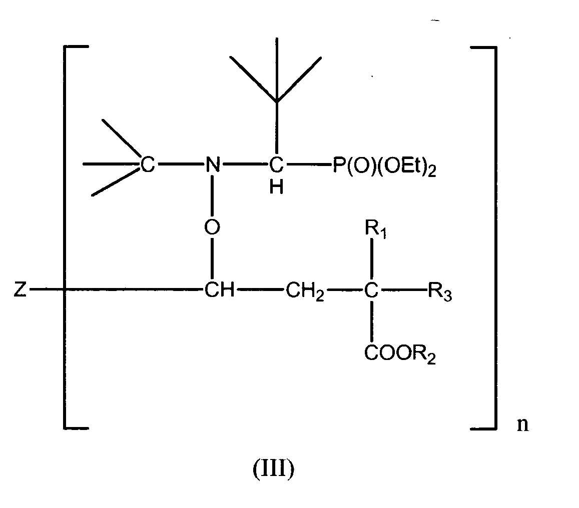 dans lesquelles :
* R₁ et R₃, identiques ou différents, représentent un groupe alkyle, linéaire ou ramifié, ayant un nombre d'atomes de carbone allant de 1 à 3 ;
* R₂ représentant un atome d'hydrogène, un groupe alkyle, linéaire ou ramifié, ayant un nombre d'atomes de carbone allant de 1 à 8, un groupe phényle, un métal alcalin, un ion ammonium;
* Z représente un groupe aryle ou un groupe de formule Z₁-[X-C(O)]ₙ, dans laquelle Z₁ représente une structure polyfonctionnelle, X est un atome d'oxygène, un atome d'azote porteur d'un groupement carboné ou d'un atome d'hydrogène, ou un atome de soufre ; et
- n est un nombre entier supérieur ou égal à 2 ;
b) une étape d'ajout au milieu réactionnel obtenu à l'issue de l'étape a) d'au moins un monomère vinylique différent de celui de l'étape a) ;
c) une étape d'ajout au milieu réactionnel d'au moins un comonomère réticulant, le comonomère réticulant étant différent des monomères des étapes a) et b), cette étape d'ajout étant réalisée simultanément ou en différé par rapport à l'étape d'ajout b),
dans lequel le ou les monomères constitutifs du macroamorceur vivant de l'étape a) et/ou les monomères vinyliques de l'étape b) et/ou le ou les comonomères réticulants de l'étape c) comprennent au moins une fonction choisie parmi -CO₂H, -SO₃H, ammonium, oxyde d'éthylène, amino, amide, ladite fonction pouvant exister éventuellement sous forme de sels.

2. Procédé selon la revendication 1, dans lequel les monomères constitutifs du macroamorceur vivant de l'étape a) comprennent au moins une fonction choisie parmi - CO₂H, -SO₃H, ammonium, oxyde d'éthylène, amino, amide, ladite fonction pouvant exister éventuellement sous forme de sels.

3. Procédé selon la revendication 1 ou 2, dans lequel les monomères constitutifs du macroamorceur vivant de l'étape a) sont choisis parmi les monomères (méth)acryliques, les monomères (méth)acrylates de dialkylaminoalkyle, les halogénures de (méth)acrylates de trialkylammoniumalkyle, les monomères (méth)acrylates d'(alcoxy)polyalkylèneglycol, les monomères (méth)acrylamides comprenant éventuellement un groupe sulfonate, les monomères styréniques comprenant un groupe sulfonate, et sont de préférence l'acide acrylique ou l'acide méthacrylique.

4. Procédé selon l'une quelconque des revendications précédentes, dans lequel l'agent de contrôle répond à la formule suivante :

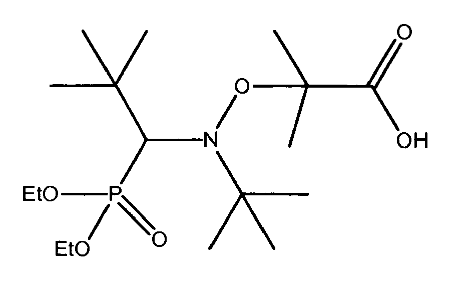

5. Procédé selon l'une quelconque des revendications 1 à 4, dans lequel l'agent de contrôle répond à la formule suivante :

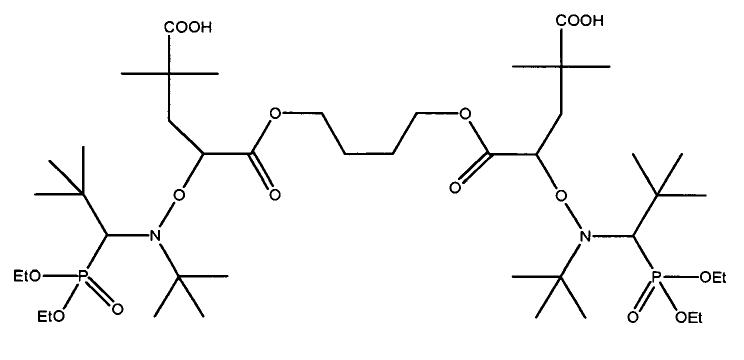

6. Procédé selon l'une quelconque des revendications précédentes, dans lequel les monomères vinyliques introduits dans le cadre de l'étape b) sont choisis parmi les monomères vinylaromatiques, les monomères acryliques tels que les acrylates d'alkyle ou d'aryle, les monomères méthacryliques tels que les méthacrylates d'alkyle ou d'aryle, et les monomères acrylamides, les monomères méthacrylamides.

7. Procédé selon l'une quelconque des revendications précédentes, dans lequel les monomères vinyliques introduits dans le cadre de l'étape b) sont des monomères monoalkylacrylamides, de préférence le N,N-diméthylacrylamide, le N,N-diéthylacrylamide ou encore le N,N-diisopropylacrylamide, ou des dialkylacrylamides, de préférence le N-méthylacrylamide, le N-éthylacrylamide, le N-isopropylacrylamide.

8. Procédé selon l'une quelconque des revendications précédentes, dans lequel les monomères vinyliques sont introduits, dans le milieu réactionnel, à raison d'au moins 10% en poids.

9. Procédé selon l'une quelconque des revendications précédentes, dans lequel les comonomères réticulants sont choisis parmi les divinylbenzènes, les trivinylbenzènes, les (méth)acrylates allyliques, les (méth)acrylates de diallylmaléatepolyol, les di(méth)acrylates d'alkylèneglycol qui ont de 2 à 10 atomes de carbone dans la chaîne carbonée, les N,N'-alkylène bisacrylamides, de préférence le N,N'-méthylène bisacrylamide.

10. Procédé selon l'une quelconque des revendications précédentes, dans lequel le comonomère réticulant est introduit, dans le milieu réactionnel, en une teneur allant de 1 à 12% en poids par rapport poids de monomère(s) vinylique(s) introduit(s) dans l'étape b).

11. Procédé selon l'une quelconque des revendications précédentes, dans lequel l'étape d'ajout c) est mise en oeuvre en différé par rapport à l'étape d'ajout b).

12. Procédé selon l'une quelconque des revendications précédentes, comprenant, en outre, une étape d'ajout au milieu réactionnel d'éléments destinés à être encapsulés par les particules.

13. Particules de microgel susceptibles d'être obtenues par un procédé tel que défini selon l'une quelconque des revendications 1 à 12.

14. Utilisation de particules de microgel telles que définies à la revendication 13 en tant que matériau d'encapsulation dans un des domaines suivants : la pharmacie, le biomédical, l'agrochimie, l'alimentation pour animaux et humains, la cosmétique, la peinture ou les revêtements de surface.

15. Utilisation de particules de microgel telles que définies à la revendication 13 en tant qu'agent épaississant dans un des domaines suivants : la pharmacie, le biomédical, l'agrochimie, l'alimentation pour animaux et humains, la cosmétique, la peinture ou les revêtements de surface.

## Claims

1. Process for preparing microgel particles in aqueous medium, comprising the following steps:
a) a step of preparing a living macroinitiator obtained by polymerization of one or more monomers in the presence of a control agent corresponding to one of the following formulae: 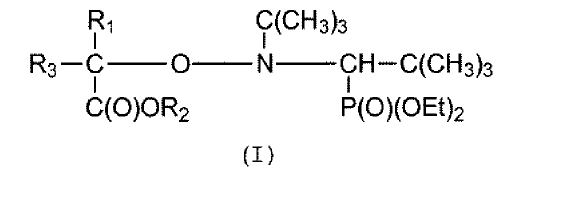 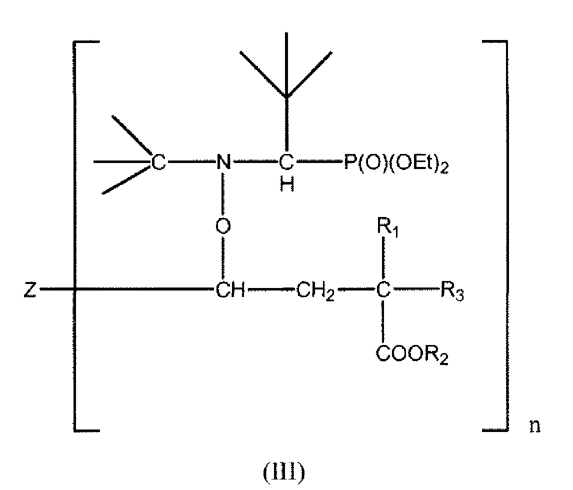 in which:
* R₁ and R₃, which may be identical or different, represent a linear or branched alkyl group having a number of carbon atoms ranging from 1 to 3;
* R₂ represents a hydrogen atom, a linear or branched alkyl group having a number of carbon atoms ranging from 1 to 8, a phenyl group, an alkali metal or an ammonium ion;
* Z represents an aryl group or a group of formula Z₁-[X-C(O)]ₙ, in which Z₁ represents a polyfunctional structure, X is an oxygen atom, a nitrogen atom bearing a carbon-based group or a hydrogen atom, or a sulfur atom; and
- n is an integer greater than or equal to 2;
b) a step of adding to the reaction medium obtained after step a) at least one vinyl monomer that is different than that of step a);
c) a step of adding to the reaction medium at least one crosslinking comonomer, the crosslinking comonomer being different than the monomers of steps a) and b), this addition step being performed simultaneously or consecutively relative to the addition step b),
in which the constituent monomer(s) of the living macroinitiator of step a) and/or the vinyl monomers of step b) and/or the crosslinking comonomer(s) of step c) comprise at least one function chosen from -CO₂H, -SO₃H, ammonium, ethylene oxide, amino and amide, said function possibly existing in the form of salts.

2. Process according to Claim 1, in which the constituent monomers of the living macroinitiator of step a) comprise at least one function chosen from -CO₂H, -SO₃H, ammonium, ethylene oxide, amino and amide, said function possibly existing in the form of salts.

3. Process according to Claim 1 or 2, in which the constituent monomers of the living macroinitiator of step a) may be chosen from (meth)acrylic monomers, dialkylaminoalkyl (meth)acrylate monomers, trialkylammoniumalkyl (meth)acrylate halides, (alkoxy)polyalkylene glycol (meth)acrylate monomers, (meth)-acrylamide monomers optionally comprising a sulfonate group, and styrene monomers comprising a sulfonate group, and preferably are acrylic acid or methacrylic acid.

4. Process according to any one of the preceding claims, in which the control agent corresponds to the following formula:

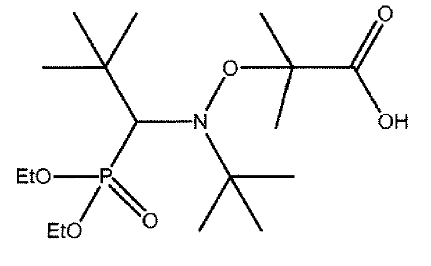

5. Process according to any one of Claims 1 to 4, in which the control agent corresponds to the following formula:

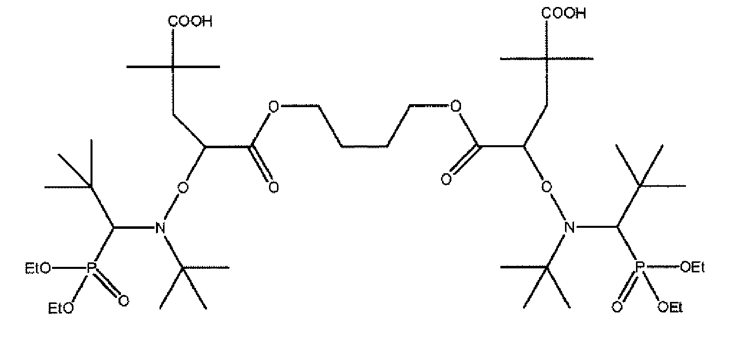

6. Process according to any one of the preceding claims, in which the vinyl monomers introduced in step b) are chosen from vinylaromatic monomers, acrylic monomers such as alkyl or aryl acrylates, methacrylic monomers such as alkyl or aryl methacrylates, acrylamide monomers and methacrylamide monomers.

7. Process according to any one of the preceding claims, in which the vinyl monomers introduced in step b) are monoalkylacrylamide monomers, preferably N,N-dimethylacrylamide, N,N-diethylacrylamide or N,N-diisopropylacrylamide or dialkylacrylamides, preferably N-methylacrylamide, N-ethylacrylamide or N-isopropylacrylamide.

8. Process according to any one of the preceding claims, in which the vinyl monomers are introduced into the reaction medium in a proportion of at least 10% by weight.

9. Process according to any one of the preceding claims, in which the crosslinking monomers are chosen from divinylbenzenes, trivinylbenzenes, allyl (meth)acrylates, diallyl maleate polyol (meth)acrylates, alkylene glycol di(meth)acrylates containing from 2 to 10 carbon atoms in the carbon chain, and N,N'-alkylenebisacrylamides, preferably N,N'-methylenebisacrylamide.

10. Process according to any one of the preceding claims, in which the crosslinking comonomer is introduced into the reaction medium in a content ranging from 1% to 12% by weight relative to the weight of vinyl monomer(s) introduced in step b).

11. Process according to any one of the preceding claims, in which the addition step c) is performed consecutively to the addition step b).

12. Process according to any one of the preceding claims, also comprising a step of adding to the reaction medium components intended to be encapsulated by the particles.

13. Microgel particles that may be obtained via a process as defined according to any one of Claims 1 to 12.

14. Use of microgel particles as defined in Claim 13, as encapsulation material in one of the following fields: pharmacy, biomedicals, agrochemistry, human or animal nutrition, cosmetics, paints or surface coatings.

15. Use of microgel particles as defined in Claim 13, as thickener in one of the following fields: pharmacy, biomedicals, agrochemistry, human or animal nutrition, cosmetics, paints or surface coatings.

## Patentansprüche

1. Verfahren zur Herstellung von Mikrogelteilchen in wäßrigem Medium mit den folgenden Schritten:
a) einem Schritt der Herstellung eines lebenden Makroinitiators, erhalten durch Polymerisation von einem oder mehreren Monomeren in Gegenwart eines Reglers einer der folgenden Formeln: 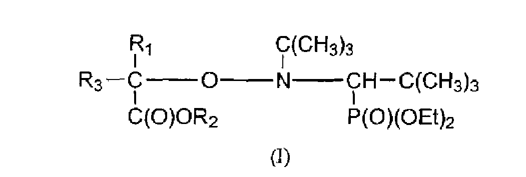 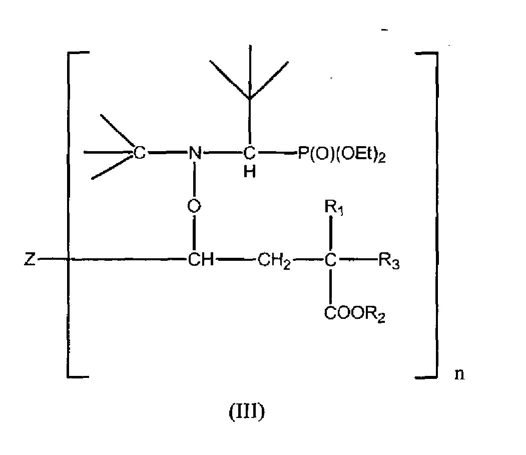 worin:
* R₁ und R₃ gleich oder verschieden sind und für eine lineare oder verzweigte Alkylgruppe mit 1 bis 3 Kohlenstoffatomen stehen;
* R₂ für ein Wasserstoffatom, eine lineare oder verzweigte Alkylgruppe mit 1 bis 8 Kohlenstoffatomen, eine Phenylgruppe, ein Alkalimetall oder ein Ammoniumion steht;
* Z für eine Arylgruppe oder eine Gruppe der Formel Z₁-[X-C(O)]ₙ, worin Z₁ für eine polyfunktionelle Struktur steht, X für ein Sauerstoffatom, ein Stickstoffatom mit einer kohlenstoffhaltigen Gruppe oder einem Wasserstoffatom oder ein Schwefelatom steht, steht und
- n für eine ganze Zahl größer gleich 2 steht;
b) einem Schritt der Zugabe mindestens eines Vinylmonomers, das von demjenigen aus Schritt a) verschieden ist, zu dem nach Schritt a) erhaltenen Reaktionsmedium;
c) einem Schritt der Zugabe mindestens eines vernetzenden Comonomers zu dem Reaktionsmedium, wobei das vernetzende Comonomer von den Monomeren der Schritte a) und b) verschieden ist, wobei dieser Zugabeschritt gleichzeitig mit oder nach dem Zugabeschritt b) durchgeführt wird,
bei dem das/die Aufbaumonomer/e des lebenden Makroinitiators von Schritt a) und/oder die Vinylmonomere von Schritt b) und/oder die vernetzenden Comonomere von Schritt c) mindestens eine unter -CO₂H, -SO₃H, Ammonium, Ethylenoxid, Amino und Amid ausgewählte Funktion umfassen, wobei die Funktion gegebenenfalls in Form von Salzen existieren kann.

2. Verfahren nach Anspruch 1, bei dem die Aufbaumonomere des lebenden Makroinitiators von Schritt a) mindestens eine unter -CO₂H, -SO₃H, Ammonium, Ethylenoxid, Amino und Amid ausgewählte Funktion umfassen, wobei die Funktion gegebenenfalls in Form von Salzen existieren kann.

3. Verfahren nach Anspruch 1 oder 2, bei dem die Aufbaumonomere des lebenden Makroinitiators von Schritt a) unter (Meth)acrylmonomeren, Dialkylaminoalkyl(meth)acrylatmonomeren, Trialkylammoniumalkyl(meth)acrylathalogeniden, (Alkoxy)-polyalkylenglykol(meth)acrylatmonomeren, (Meth)-acrylamidmonomeren, die gegebenenfalls eine Sulfonatgruppe umfassen, und Styrolmonomeren mit einer Sulfonatgruppe ausgewählt werden und vorzugsweise Acrylsäure oder Methacrylsäure sind.

4. Verfahren nach einem der vorhergehenden Ansprüche, bei dem der Regler der folgenden Formel entspricht:

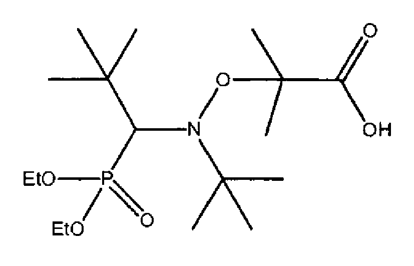

5. Verfahren nach einem der Ansprüche 1 bis 4, bei dem der Regler der folgenden Formel entspricht:

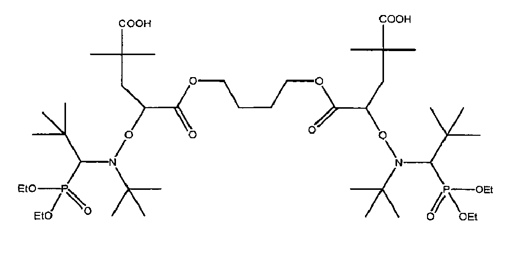

6. Verfahren nach einem der vorhergehenden Ansprüche, bei dem die im Rahmen von Schritt b) eingetragenen Vinylmonomere unter vinylaromatischen Monomeren, Acrylmonomeren wie Alkyl- oder Arylacrylaten, Methacrylmonomeren wie Alkyl- oder Arylmethacrylaten, Arylamidmonomeren und Methacrylamidmonomeren ausgewählt werden.

7. Verfahren nach einem der vorhergehenden Ansprüche, bei dem es sich bei den im Rahmen von Schritt b) eingetragenen Vinylmonomeren um Monoalkylacrylamidmonomere, vorzugsweise N,N-Dimethylacrylamid, N,N-Diethylacrylamid oder auch N,N-Diisopropylacrylamid, oder Dialkylacrylamide, vorzugsweise N-Methylacrylamid, N-Ethylacrylamid oder N-Isopropylacrylamid, handelt.

8. Verfahren nach einem der vorhergehenden Ansprüche, bei dem man die Vinylmonomere in einem Anteil von mindestens 10 Gew.-% in das Reaktionsmedium einträgt.

9. Verfahren nach einem der vorhergehenden Ansprüche, bei dem man die vernetzenden Comonomere unter Divinylbenzolen, Trivinylbenzolen, Allyl(meth)-acrylaten, Diallylmaleatpolyol(meth)acrylaten, Alkylenglykoldi(meth)acrylaten mit 2 bis 10 Kohlenstoffatomen in der Kohlenstoffkette und N,N'-Alkylenbisacrylamiden, vorzugsweise N,N'-Methylenbisacrylamid, auswählt.

10. Verfahren nach einem der vorhergehenden Ansprüche, bei dem man das vernetzende Comonomer in einem Gehalt von 1 bis 12 Gew.-%, bezogen auf das Gewicht des bzw. der in Schritt b) eingetragenen Vinylmonomere, in das Reaktionsmedium einträgt.

11. Verfahren nach einem der vorhergehenden Ansprüche, bei dem man den Zugabeschritt c) nach dem Zugabeschritt b) durchführt.

12. Verfahren nach einem der vorhergehenden Ansprüche, das außerdem einen Schritt der Zugabe von zur Verkapselung durch die Teilchen bestimmten Elementen zum Reaktionsmedium umfaßt.

13. Mikrogelteilchen, die nach einem Verfahren gemäß einem der Ansprüche 1 bis 12 erhältlich sind.

14. Verwendung von Mikrogelteilchen gemäß Anspruch 13 als Verkapselungsmaterial auf einem der folgenden Gebiete: Pharmazie, Biomedizin, Agrochemie, Tierfutter, Lebensmittel, Kosmetik, Anstrichmittel oder Lacke.

15. Verwendung von Mikrogelteilchen gemäß Anspruch 13 als Verdickungsmittel auf einem der folgenden Gebiete: Pharmazie, Biomedizin, Agrochemie, Tierfutter, Lebensmittel, Kosmetik, Anstrichmittel oder Lacke.
